(19) Europäisches Patentamt
European Patent Office
Office européen des brevets

(11) **EP 3 338 811 A1**

(12) **EUROPÄISCHE PATENTANMELDUNG**

(43) Veröffentlichungstag:
**27.06.2018  Patentblatt 2018/26**

(51) Int Cl.:
***A61L 2/28*** (2006.01)  ***C12M 1/34*** (2006.01)
***C12Q 1/22*** (2006.01)

(21) Anmeldenummer: **17207058.3**

(22) Anmeldetag: **13.12.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(30) Priorität: **14.12.2016  DE 102016124284**

(71) Anmelder: **Compliance Advice and Services in Microbiology GmbH**
**50739 Köln (DE)**

(72) Erfinder:
• **Flier, Niwin**
  **52351 Düren (DE)**
• **Haberer, Dr. Klaus**
  **65719 Hofheim (DE)**
• **Böker, Prof. Dr. Alexander**
  **13156 Berlin (DE)**

(74) Vertreter: **Cohausz & Florack**
**Patent- & Rechtsanwälte**
**Partnerschaftsgesellschaft mbB**
**Bleichstraße 14**
**40211 Düsseldorf (DE)**

(54) **ÜBERPRÜFUNG EINER STERILISATIONSWIRKUNG**

(57)  Die Erfindung betrifft eine Vorrichtung zur Überprüfung einer Sterilisationswirkung, umfassend:
ein Tragelement, wobei mindestens zwei Teilbereiche der Oberfläche des Tragelementes bindungsfähig sind und wobei die bindungsfähigen Teilbereiche voneinander beabstandet sind,
einen Haftvermittler, wobei die bindungsfähigen Teilbereiche der Oberfläche durch die Anordnung des Haftvermittlers an diesen funktionalisierbar sind, wobei der Haftvermittler einerseits an dem bindungsfähigen Teilbereich der Oberfläche anbindet und andererseits durch die jeweilige Größe des bindungsfähigen Teilbereichs bedingt freie Haftstellen für anzuheftende Mikroorganismen bereitstellt, so dass zwischen dem bindungsfähigen Teilbereich der Oberfläche des Tragelementes und anzuheftenden Mikroorganismen eine stabile vermittelnde Bindung herstellbar ist, und
eine Population von Mikroorganismen, wobei die Mikroorganismen der Population gereinigt sind, wobei an den Mikroorganismen anhaftendes überschüssiges biologisches Material entfernt ist und die jeweiligen Mikroorganismen quasi Einzelzellcharakter aufweisen,
wobei jeweils eine gereinigte Population von Mikroorganismen an einem der funktionalisierten Teilbereiche der Oberfläche derart anheftbar ist, dass die Mikroorganismen in einer hohen Flächendichte und im Wesentlichen ohne gegenseitige Überdeckung in einer Einzelschicht angeheftet sind.

Fig. 1

**Beschreibung**

**Gebiet**

Die Erfindung betrifft eine Vorrichtung zur Überprüfung einer Sterilisationswirkung. Darüber hinaus betrifft die Erfindung ein Verfahren zu dessen Herstellung und eine Verwendung der erfindungsgemäßen Vorrichtung.

**Hintergrund**

Produkte die steril sein müssen, um ihre Anwendungssicherheit (z.B. bei sterilen Arzneimitteln und Medizinprodukten), oder ihre Haltbarkeit (z.B. bei Konserven) sicherzustellen, müssen geeigneten Sterilisationsverfahren unterzogen werden. Je nach Eigenschaften des Sterilisierguts kommen unterschiedliche Prinzipien zur Anwendung, wie beispielsweise eine Exposition in gesättigtem Wasserdampf, bei trockener Hitze oder unter ionisierender Bestrahlung. Ferner wird eine chemische Sterilisation vor allem zur Sterilisation von Oberflächen eingesetzt, z. B. mittels Wasserstoffperoxid ($H_2O_2$)-Dampf in Isolatoren oder mittels Begasen mit Ethylenoxid ($C_2H_4O$) bei medizinischen Geräten und Einmalartikeln.

Zur Überprüfung der biologischen Wirksamkeit von Sterilisationsverfahren werden als Bioindikatoren in der Regel Dauerformen, beispielsweise Sporen bestimmter Mikroorganismen mit hoher Resistenz gegenüber dem Sterilisationsverfahren verwendet. Die Resistenz der Mikroorganismen ist immer nur gegenüber einem Sterilisationsverfahren bestimmbar und wird als dezimale Reduktionszeit (D-Wert) angegeben. Dieser D-Wert ist die gleichbleibende Zeit, in der eine derart behandelte Population unter den gegebenen Sterilisationsbedingungen um 90% verringert wird. Einerseits ist der D-Wert eine spezifische Eigenschaft des Stamms der Mikroorganismen, beispielsweise einem Bakterienstamm, wobei Anzucht, Sporulation und Präparation der Sporen von den Mikroorganismen nicht nur den D-Wert jeder Charge modulieren, sondern auch die Widerstandsfähigkeit der einzelnen Mikroorganismen einer Schwankungsbreite unterliegt, die vom Herstellverfahren abhängig ist. Daher sind D-Werte zwangsläufig mit einer biologischen Unschärfe behaftet. Andererseits ändert sich die Reduktionszeit mit unterschiedlicher Wirksamkeit des Sterilisationsverfahrens. Ein angegebener D-Wert gilt somit immer nur für die zugehörigen Sterilisationsbedingungen.

Zur Prüfung der Sterilisationswirkung werden Populationen von Mikroorganismen in hoher Anzahl auf geeignete Träger aufgebracht. Die derart beaufschlagten Träger werden dann, mit oder ohne Sterilisiergut, in einer Sterilisierkammer den zu überprüfenden Sterilisationsbedingungen ausgesetzt. Aus der Kenntnis der Zahl der Mikroorganismen auf dem Träger vor der Exposition und der Zahl der überlebenden Mikroorganismen nach der Exposition lässt sich der D-Wert berechnen. In der Praxis ist die Bestimmung der expliziten Zahl überlebender Mikroorganismen zwar möglich, aber mit hohem Aufwand verbunden, und durch die erforderliche Wiedergewinnung der Mikroorganismen auch fehleranfällig. Daher wird die mittlere Zeit bis zur vollständigen Inaktivierung aller Sporen der ursprünglichen Populationen auf jedem der eingesetzten Träger abgeschätzt.

Zur Auswertung werden die Träger nach Exposition gegenüber Sterilisationsbedingungen mit einem Nährmedium in Kontakt gebracht, und anschließend zuvor definierten Wachstumsbedingungen ausgesetzt. Dabei sollen die letzten noch überlebenden Sporen der Mikroorganismen-Population erkennbar gemacht werden. Eine verlängerte Zeit bis zur vollständigen Abtötung zeigt eine geringere Wirksamkeit des Sterilisationsverfahrens an, und umgekehrt. Ein erhebliches Problem bei dieser Auswertung besteht in der Art der Absterbekurve, die sich als Kinetik erster Ordnung dem Nullwert asymptotisch annähert. Der Zeitpunkt der vollständigen Inaktivierung ist daher nicht exakt bestimmbar, sondern bedarf der Abschätzung mit Hilfe statistischer Verfahren. Die tatsächliche Ausgangszahl von Mikroorganismen auf einem Träger, sowie die unvermeidliche biologische Unschärfe des D-Werts machen eine statistische Herangehensweise zwingend erforderlich.

Bei den gegenwärtig bekannten Bioindikatoren ist angegeben, dass auf jedem Träger eine einzige Population von etwa 1 x $10^6$ Mikroorganismen angeordnet ist. Diese Population von Mikroorganismen nimmt den ganzen oder den größten Teil der gesamten Trägeroberfläche ein (Beispiele sind Papierstreifen von etwa 3 x 0,5 mm oder 3 x 0,5 cm für Dampfsterilisation oder runde Stahlträger mit etwa 0,8 cm Durchmesser für Gassterilisation). Der ISO Standard fordert, dass die Herstellerangabe für die Anzahl von Mikroorganismen pro Träger zwischen -50% und + 300% reproduzierbar sein soll. Ferner soll der angegebene D-Wert unter Anwendung der Methode des Herstellers dieses Bioindikators mit ±20% reproduzierbar sein. Es ist offensichtlich, dass die mit diesen Spannen verbundene Unschärfe groß ist.

Das Überleben einzelner Sporen nach einer erfolgten Exposition gegenüber Sterilisationsbedingungen kann daher sowohl aus der Variabilität der biologischen Indikatoren, als auch aus einer zu geringen Wirkung des Sterilisationsverfahrens erklärbar sein.

Es werden nach Ph. Eur. (*Pharmacopoea Europaea,* Europäisches Arzneibuch) Bioindikatoren vorgeschlagen, bei denen die Wahrscheinlichkeit nach Exposition gegenüber Sterilisationsbedingungen eines Standard-Sterilisationsverfahrens überlebende Zellen zu finden, bei 1 in $10^4$ liegt. Sind nach der Exposition überlebende Mikroorganismen vorhanden, gilt dies als Hinweis auf eine unzureichende Sterilisation, sind hingegen alle Mikroorganismen vollständig abgetötet, bleibt die Aussage über die korrekte Wirksamkeit des Prozesses dennoch schwach, da lediglich erfassbar ist, dass alle Mikroorganismen abgetötet wurden, nicht jedoch beispielsweise wie lange dies gedauert oder wie resistent die Mikroorganismen gegenüber den Sterilisationsbedingungen waren.

**Zusammenfassung einiger beispielhafter Ausführungsformen**

Ausgehend von diesem Stand der Technik besteht die Aufgabe der Erfindung darin, eine Vorrichtung Überprüfung einer Sterilisationswirkung bereitzustellen, die die vorstehend angeführten Probleme verbessert und die es insbesondere ermöglicht, eine statistisch relevante Auswertung durchzuführen.

Zur technischen Lösung dieser Aufgabe wird mit der Erfindung eine Vorrichtung zur Überprüfung einer Sterilisationswirkung vorgeschlagen, umfassend:

ein Tragelement, wobei mindestens zwei Teilbereiche der Oberfläche des Tragelementes bindungsfähig sind und wobei die bindungsfähigen Teilbereiche voneinander beabstandet sind,

einen Haftvermittler, wobei die bindungsfähigen Teilbereiche der Oberfläche durch die Anordnung des Haftvermittlers an diesen funktionalisierbar sind, wobei der Haftvermittler einerseits an dem bindungsfähigen Teilbereich der Oberfläche anbindet und andererseits durch die jeweilige Größe des bindungsfähigen Teilbereichs bedingt freie Haftstellen für anzuheftende Mikroorganismen bereitstellt, so dass zwischen dem bindungsfähigen Teilbereich der Oberfläche des Tragelementes und anzuheftenden Mikroorganismen eine stabile vermittelnde Bindung herstellbar ist, und

eine Population von Mikroorganismen, wobei die Mikroorganismen der Population gereinigt sind, wobei an den Mikroorganismen anhaftendes überschüssiges biologisches Material entfernt ist und die jeweiligen Mikroorganismen quasi Einzelzellcharakter aufweisen,

wobei jeweils eine gereinigte Population von Mikroorganismen an einem der funktionalisierten Teilbereiche der Oberfläche derart anheftbar ist, dass die Mikroorganismen in einer hohen Flächendichte und im Wesentlichen ohne gegenseitige Überdeckung in einer Einzelschicht angeheftet sind.

Der Erfindung liegt zum einen die Erkenntnis zugrunde, dass eine Anheftung von Mikroorganismen an einem funktionalisierten Teilbereich der Oberfläche des Tragelements eine im definierte und reproduzierbare Anzahl von Mikroorganismen pro funktionalisierten Teilbereich ermöglicht. Die Anheftung der Mikroorganismen im Wesentlichen ohne gegenseitige Überdeckung ermöglicht eine gleichförmige Exposition der einzelnen Mikroorganismen einer Population von Mikroorganismen gegenüber Sterilisationsbedingungen. Es ist ein gleichförmiger Zutritt sterilisierenden Agens sowohl beispielsweise für kondensierenden Wasserdampf, als auch zum Beispiel für dampfförmiges Wasserstoffperoxid möglich. Dies ermöglicht eine einheitliche Exposition aller auf einer Ausgestaltung einer erfindungsgemäßen Vorrichtung anheftbaren Mikroorganismen. Die anheftbaren Mikroorganismus-Population ist auf die zu charakterisierende Sterilisationswirkung in Anzahl und Resistenz derart optimiert, dass bei einer erwarteten Sterilisationswirkung alle oder nahezu alle Populationen der auf dem Tragelement anheftbaren Populationen vollständig abgetötet werden. Eine verminderte Sterilisationswirkung wird durch das Überleben von Mikroorganismen einer einzelnen Population von Mikroorganismen der Ausgestaltung einer erfindungsgemäßen Vorrichtung angezeigt. Durch die erfindungsgemäß hohe Flächendichte in einer Populationen von Mikroorganismen in Verbindung mit der Resistenz der Präparation vor der Anheftung der Mikroorganismen kann die Belastung der Sterilisationswirkung entsprechend gewählt werden, um eine statistisch signifikante Aussage zu treffen.

Zum anderen liegt der Erfindung die Erkenntnis zugrunde, dass die statistische Auswertung der von mehreren Populationen von Mikroorganismen wesentlich vereinfacht ist, wenn mehrere Populationen von Mikroorganismen mit nur einer Vorrichtung bzw. nur einem Tragelement gleichzeitig gegenüber Sterilisationsbedingungen exponiert werden können. Erfindungsgemäß ist also vorgesehen, dass mindestens zwei Populationen von Mikroorganismen auf nur einem Tragelement angeordnet bzw. angeheftet sind. Dies erlaubt eine statistische Auswertung der Sterilisation bei der Verwendung nur einer erfindungsgemäßen Vorrichtung.

Die Mikroorganismen einer Population sind in einer Ausgestaltung nach allen vorgenannten Aspekten der Erfindung definiert anheftbar auf einem bindungsfähigen und funktionalisierten Teilbereich auf der Oberfläche des Tragelementes, wobei eine Trennung von einzelnen Populationen auf nur einem Tragelement möglich ist. Hierdurch sind die herrschenden Sterilisationsbedingungen populationsübergreifend im Wesentlichen einheitlich, wodurch eine weit verbesserte statistische Auswertung ermöglicht ist, wobei falsch positive bzw. falsch negative Ausreißer im Auswertungsergebnis minimiert werden.

Im Sinne der vorliegenden Erfindung wird unter dem Begriff Haftvermittler eine Substanz verstanden, die zwischen zwei unmischbaren Stoffen eine Bindung ermöglicht. Der Haftvermittler wird auf einer Oberfläche eines ersten Stoffes angeordnet und ermöglicht zwischen dem ersten Stoff und einem weiteren zweiten Stoff eine enge, stabile und dauerhafte Bindung.

Eine Population von Mikroorganismen umfasst im Sinne der Erfindung eine Anzahl Mikroorganismen, die eine Resistenz gegenüber einem Sterilisationsprinzip und den damit verbundenen Sterilisationsbedingungen aufweisen. Es können Mikroorganismus-Populationen mit einer Anzahl von etwa $10^4$, etwa $10^5$, etwa $10^6$, etwa $10^7$ oder etwa $10^8$ Mikroorganismen pro Population auf einem bindungsfähigen und funktionalisierten Teilbereich der Oberfläche der erfindungsgemäßen Vorrichtung angeheftet werden.

Vorzugsweise weist das Tragelement zwei bis 96, bevorzugt zwei, vier, sechs, acht, zehn, zwölf, 20, 21, 22, 24, 27, 30, 40, 48 oder 96 aktivierte und/oder funktionalisierte Teilbereiche zur Anheftung von Mikroorganismen auf. Das Tragelement kann also mehr als zwei aktivierte Teilbereiche aufweisen. Die Zahl der Populationen auf aktivierten Teiloberflächen beträgt erfindungsgemäß mindestens 2, eine höhere Zahl an Populationen erlaubt verbesserte statistische Auswertung. Die Obergrenze der Populationszahl ist gegeben durch den beschriebenen Platzbedarf der Einzelpopulation zuzüglich einer ausreichenden physikalischem Distanz, zwischen allen Populationen die eine unabhängige Auswertung aller Einzelpopulationen ermöglicht. Die Anzahl der bindungsfähigen Teilbereiche auf der Oberfläche des Tragelementes ist begrenzt durch i) die Größe des Tragelementes, ii) die für eine aussagekräftige Auswertbarkeit erforderliche Größe einer Einzelpopulationen, iii) durch die erreichbare Flächendichte bei der Anheftung von Mikroorganismen, die sich unmittelbar auf die Größe des benötigten bindungsfähigen Teilbereichs auswirkt, sowie iv) der Anordnung von Populationen auf dem Tragelement unter Berücksichtigung der Trennung der einzelnen Populationen von Mikroorganismen. Multiple Populationen auf nur einem einzigen Tragelement der Vorrichtung ermöglichen eine gemeinsame Exposition gegenüber während eines Sterilisationsvorgangs auftretenden Sterilisationsbedingungen. Das Auswertungsergebnis im Rahmen der statistischen Auswertung ist durch die gemeinsame Exposition der multiplen Populationen wesentlich unempfindlicher gegenüber Ausreißern, da die herrschenden Sterilisationsbedingungen wesentlich konstanter sind.

Die Beabstandung der mindestens zwei bindungsfähigen Teilbereiche erfolgt über die Herstellung einer physikalischen Distanz zwischen diesen bindungsfähigen Teilbereichen der Oberfläche des Tragelementes, wobei zwischen zwei bindungsfähigen Teilbereichen der Oberfläche des Tragelementes keine Berührungspunkte bestehen dürfen. Die physikalische Distanz ist vorteilhafterweise so groß, dass die Lebensfähigkeit einzelner Mikroorganismen in den auf den bindungsfähigen Teilbereichen anhaftenden Mikroorganismen-Populationen getrennt und unabhängig voneinander erkennbar gemacht werden kann. Ansonsten können sich einzelne Mikroorganismus-Populationen gegenseitig kontaminieren, so dass nach der Durchführung einer Sterilisation einzelne überlebende Mikroorganismen der jeweiligen Population nicht sicher zugeordnet werden können. Ist beispielsweise nach der Durchführung einer Sterilisation eine Population von Mikroorganismen vollständig abgetötet, und eine weitere Population hingegen nicht, könnten bei einer Verbindung zwischen den beiden Mikroorganismen-Populationen einzelne Mikroorganismen "überwandern", so dass eine sichere Zuordnung der einzelnen überlebenden Mikroorganismen zu ihrer jeweiligen Population nicht möglich ist. Dies führt zu einem falsch positiven bzw. falsch negativen statistischen Auswertungsergebnis.

Vorteilhafterweise ist die Fläche der bindungsfähigen Teilbereiche der Oberfläche jeweils vollständig und gleichmäßig mit einem Haftvermittler bedeckt, so dass an der gesamten Fläche des jeweiligen bindungsfähigen Teilbereichs der Oberfläche des Tragelementes Mikroorganismen anbinden können. Dabei bindet ein bindungsfähiger Teilbereich den Haftvermittler in einer so hohen Flächendichte, dass an dem gesamten bindungsfähigen Teilbereich Mikroorganismen in der durch die freien Haftstellen vorbestimmten Dichte an dem Tragelement anheftbar sind. Die Anzahl der freien Haftstellen, auch Bindungsstellen genannt, kann einerseits durch die Fläche eines bindungsfähigen Teilbereichs andererseits durch die Flächendichte des Haftvermittlers moduliert werden.

In einer Ausgestaltung der vorliegenden Erfindung nach allen vorgenannten Aspekten erfolgt die Anheftung von Mikroorganismen im Wesentlichen, insbesondere ausschließlich an den bindungsfähigen und funktionalisierten Teilbereichen der Oberfläche des Tragelementes. Dadurch ist die Anheftung von mindestens zwei Populationen von Mikroorganismen auf nur einem einzigen Tragelement gemäß einer Ausgestaltung einer erfindungsgemäßen Vorrichtung nach allen vorgenannten Aspekten möglich.

Erfindungsgemäß werden anzuheftende Mikroorganismen vor deren Anheftung auf einem Tragelement nach einer der vorstehenden Ausgestaltung der Vorrichtung gereinigt. Bei dieser Reinigung, auch Aufreinigung genannt, werden die Mikroorganismen von biologischem Material, wie beispielsweise Nährmedienbestandteile oder Zelltrümmer, die von der Anzucht der Mikroorganismen herrühren, abgetrennt. Die Reinigung umfasst beispielsweise Waschvorgänge und Zentrifugationsschritte, die beispielsweise eine chemische und/oder physikalische Reinigung ermöglichen. Im Nachfolgenden ist eine mögliche Reinigung von Mikroorganismen aufgeführt. Erfindungsgemäß ist eine hohe Aufreinigung der anzuheftenden Mikroorganismen vorgesehen, wobei eine derartige hoch gereinigte Präparation von Mikroorganismen so intensiv gereinigt sind, dass auch lose an der Oberfläche der Mikroorganismen anhaftende Fremdbestandteile abgelöst sind. Dieses Merkmal eines derart gereinigten Mikroorganismus wird im Sinne der Erfindung unter einem Mikroorganismus, der Einzelzellcharakter aufweist, aufgefasst. Die Resistenzeigenschaften der Mikroorganismen gegenüber einer Sterilisation bleiben dabei erhalten, wobei dies durch eine entsprechende Überprüfung sichergestellt werden kann.

Dies ermöglicht, dass im Wesentlichen nur die Anheftung der hoch gereinigten Mikroorganismen an den freien Haftstellen des Haftvermittlers erfolgt. Eine Verklumpung und/oder sekundäre Anlagerung einzelner Mikroorganismen, insbesondere bedingt durch Querverknüpfung über kontaminierendes biologisches Material wird vermieden. Dadurch unterstützt ferner die Anheftung in hoher Flächendichte auf den bindungsfähigen und funktionalisierten Teilbereichen der Oberfläche des Tragelementes.

Eine bevorzugte Ausgestaltung der Erfindung ist dadurch gekennzeichnet, dass die hohe Flächendichte der auf den funktionalisierten Teilbereichen der Oberfläche des Tragelementes anheftbaren Mikroorganismen einem Wert entspricht, dessen Standardabweichung etwa 10% oder weniger bezogen auf eine Flächendichte von etwa $10^6$ Mikroorganismen pro 5 mm$^2$ beträgt. Vorteilhafterweise beträgt diese Standardabweichung etwa 9% oder weniger, etwa 8 % oder weniger, etwa 7% oder weniger, etwa 6% oder weniger, etwa 5% oder weniger, etwa 4% oder weniger, bevorzugt etwa 3% oder weniger, besonders bevorzugt etwa 2% oder weniger oder etwa 1% oder weniger bezogen auf eine Flächendichte von etwa $10^6$ Mikroorganismen pro 5 mm$^2$. Wird eine Population von Mikroorganismen auf einem bindungsfähigen und funktionalisierten Teilbereich der Oberfläche des Tragelementes angeheftet, deren absolute Individuenanzahl von Mikroorganismen von etwa $10^6$ Mikroorganismen abweicht, verändert sich nach dem angegeben Verhältnis von etwa $10^6$ Mikroorganismen pro 5 mm$^2$ zwar deren benötige Fläche, die Flächendichte jedoch entspricht weiterhin einem Wert, dessen Standardabweichung etwa 10% oder weniger bezogen auf eine Flächendichte von etwa $10^6$ Mikroorganismen pro 5 mm$^2$ beträgt, wobei vorstehend angeführte weitere Standardabweichungen ebenfalls möglich sind. Die maximal erreichbare Flächendichte bei einer Anheftung von Mikroorganismen auf einer Ausgestaltung nach allen vorgenannten Aspekten der vorliegenden Erfindung beträgt bei durchgehend einzelliger parakristalliner Anordnung $10^6$ Mikroorganismen pro mm$^2$.

In einer Ausgestaltung der Erfindung erlaubt die Anheftung von Mikroorganismen auf dem Trageelement, die Belastung durch Mikroorganismen, die im Prozess anforderungsgemäß inaktiviert werden sollen, prozessentsprechend zu variieren. Höhere Resistenz oder eine größere Zahl der eingesetzten Mikroorganismen erlaubt die aussagekräftige Prüfung höher wirksamer Prozesse, eine höhere Anzahl an Populationen von Mikroorganismen je Tragelement ermöglicht eine genauere statistische Auswertung. In einer weiteren Ausgestaltung der Erfindung wird die Anzahl von Mikroorganismen aller Einzelpopulationen auf einem Tragelement gleich, wobei die Standardabweichung $\pm$10% oder weniger, vorzugsweise beträgt die Standardabweichung $\pm$9%, $\pm$8%, $\pm$7%, $\pm$6%, $\pm$5% oder weniger.

Die Anforderungen für die Inaktivierung von auf einer erfindungsgemäßen Vorrichtung anheftbaren Mikroorganismen ergibt sich aus der Kombination von Anzahl Mikroorganismen pro Population, Resistenz der jeweiligen Mikroorganismen und Anzahl der eingesetzten Populationen von Mikroorganismen, also der jeweils auf einem funktionalisierten Teilbereich einer erfindungsgemäßen Vorrichtung anheftbaren Populationen von Mikroorganismen. Bei einem Sterilisationsprozess, dessen erwartete Sterilisationswirkung die eingesetzten Mikroorganismen um 8 logarithmische Größenordnungen abtöten, d.h. inaktivieren soll, weist das Trageelement beispielsweise 10 Populationen mit je $10^6$ Sporen auf. Die Gesamtzahl der Mikroorganismen auf dem Träger beträgt dann $10^7 = 10 \times 10^6$. In der statistischen Erwartung beträgt die Überlebenswahrscheinlichkeit $10^{-1}$, somit wird theoretisch nur eines von 10 Trageelementen noch eine überlebende Spore aufweisen.

Ist die Sterilisationswirkung reduziert, so dass nur 7 Größenordnungen abgetötet werden, so ist die Überlebenswahrscheinlichkeit $10^0 = 1$, das heißt, dass etwa bei 60 % aller Tragelemente Populationen mit überlebenden Mikroorganismen gefunden werden. Werden durch den Prozess nur 6 Größenordnungen abgetötet, so ist die Überlebenswahrscheinlichkeit in jeder Population $10^0$, somit werden in ca. 60% aller Populationen überlebende Mikroorganismen erwartet. Bei 100 Populationen mit je $10^5$ Sporen pro Trageelement ist die Aussagefähigkeit des Bioindikators über die Wirksamkeit des Prozesses ebenso hoch, die statistische Aussage wird jedoch genauer.

Exemplarisch sei an dieser Stelle folgender Vergleich zum Stand der Technik angeführt: Aus dem Stand der Technik ist es bekannt, $10^6$ Mikroorganismen auf einem Papierstreifen der Größe 3 cm x 0,5 cm = 1,5 cm$^2$ = 150 mm$^2$ anzuordnen. Dies entspricht etwa $10^6$ Mikroorganismen pro 150 mm$^2$, also etwa 6667 Mikroorganismen pro mm$^2$.

Ferner ist es bekannt, etwa $10^6$ Mikroorganismen auf einem runden Stahlträger mit einem Durchmesser von etwa 0,8 cm anzuordnen. Dies entspricht einer Fläche von etwa $(0,8 \text{ cm} \div 2)^2 \times \pi = 0,5 \text{cm}^2 = 50 \text{mm}^2$, also etwa $10^6$ Mikroorganismen pro 50mm$^2$ = 20000 Mikroorganismen pro mm$^2$.

Die erfindungsgemäße Vorrichtung ermöglicht es, $10^6$ Mikroorganismen pro 5 mm$^2$, also etwa 200000 Mikroorganismen pro mm$^2$ anzuordnen.

Eine vorteilhafte Ausgestaltung der Erfindung sieht vor, dass die Anheftung von Mikroorganismen in der Einzelschicht derart erfolgt, wobei etwa 10% oder weniger der Mikroorganismen sich gegenseitig überdecken. Vorzugsweise beträgt die Standardabweichung der Anzahl von sich gegenseitig überdeckenden Mikroorganismen etwa 10% oder weniger bezogen auf eine Flächeneinheit des bindungsfähigen Teilbereichs der Oberfläche im Vergleich zu einer weiteren Flächeneinheit des bindungsfähigen Teilbereichs der Oberfläche. Vorteilhafterweise überdecken sich etwa 9% oder weniger, etwa 8 % oder weniger, etwa 7% oder weniger, etwa 6% oder weniger, etwa 5% oder weniger, etwa 4% oder weniger, bevorzugt etwa 3% oder weniger, besonders bevorzugt etwa 2% oder weniger oder etwa 1% oder weniger der Mikroorganismen gegenseitig von einer Population von Mikroorganismen.

Eine vorteilhafte Ausgestaltung der Erfindung sieht vor, dass die Anheftung von Mikroorganismen im Wesentlichen ohne gegenseitige Überdeckung in einer quasi Monoschicht erfolgt, wobei nicht mehr als etwa 10% der Mikroorganismen sich gegenseitig überdecken. Vorteilhafterweise überdecken sich weniger oder keine der ausgewerteten Mikroorganismen. Dies wird beispielsweise durch eine elektronenmikroskopische Auswertung von nicht weniger als 200 Mikroorganismen im Randbereich repräsentativer aktivierter Oberflächen bestätigt.

Die Anordnung in einer quasi Monoschicht, auch Monolayer oder Einzellage genannt, in überwiegend einlagiger Schicht wird durch eine Anheftung der Mikroorganismen an dem Tragelement über den Haftvermittler ermöglicht, wobei die Mikroorganismen dabei insbesondere nahezu ausschließlich bzw. ausschließlich über die vermittelnde Bindung des Haftvermittlers an dem bindungsfähigen Teilbereich des Tragelementes angeheftet werden. Geeignete und ausreichende Waschverfahren der mit Mikroorganismen beaufschlagten Oberflächen entfernen sekundär angelagerte Mikroorganismen, während die mittels Haftvermittler an dem aktivierten Teilbereich dauerhaft angehefteten Mikroorganismen nicht abgewaschen bzw. abgelöst werden. Nach dem Waschverfahren bleiben im Wesentlichen, insbesondere ausschließlich nur noch die über den Haftvermittler an den bindungsfähigen Teilbereichen angehefteten Mikroorganismen übrig, die nicht abwaschbar sind. Dies vermindert die Überdeckung bzw. das Verklumpen von Mikroorganismen wesentlich. In dem so entstehenden Monolayer wird ein gegenseitiger Schutz der Mikroorganismen vor den Sterilisationsbedingungen weitestgehend vermieden, bzw. die Möglichkeit des gegenseitigen Schutzes der Mikroorganismen wird minimiert.

Eine weitere Ausgestaltung der Erfindung ist dadurch gekennzeichnet, dass die mindestens zwei bindungsfähigen Teilbereiche der Oberfläche des Tragelementes aktiviert sind.

Unter dem Begriff Aktivierung einer Oberfläche wird im Sinne der vorliegenden Erfindung die mechanische, thermische und/oder chemische Behandlung der Oberfläche oder eine gleichartige Behandlung der Oberfläche aufgefasst.

Die Aktivierung wenigstens eines Teilbereichs der Oberfläche von dem Tragelement wird vorteilhafterweise durch eine Plasmabehandlung erreicht. Hierdurch wird die Oberflächenspannung an den bindungsfähigen Teilbereichen erhöht. Zur Aktivierung nur eines Teilbereichs der Oberfläche des Tragelementes wird der nicht zu aktivierende Teil der Oberfläche gegenüber dem Zutritt von Plasma abgeschirmt.

Eine Ausgestaltung der Erfindung sieht vor, dass die jeweilige Fläche der bindungsfähigen Teilbereiche auf der Oberfläche des Tragelementes im Wesentlichen gleich groß ist. Zudem können die bindungsfähigen Teilbereiche neben einer identischen Flächengröße auch eine identische Form aufweisen. Durch die gleiche Größe der bindungsfähigen Teilbereiche stellen diese nach der Funktionalisierung mit einem Haftvermittler im Wesentlichen die gleiche Anzahl an freien Haftstellen für anzuheftende Mikroorganismen bereit. So kann sichergestellt werden, dass auf jedem bindungsfähigen und mit dem Haftvermittler funktionalisierten Teilbereich auf der Oberfläche des Tragelementes im Wesentlichen die gleiche Individuenzahl von Mikroorganismen angeheftet werden kann.

In einer weiteren Ausgestaltung der Erfindung bindet der Haftvermittler elektrostatisch oder kovalent an den bindungsfähigen Teilbereichen der Oberfläche des Tragelementes an. Eine weitere Ausgestaltung der Erfindung sieht vor, dass anzuheftende Mikroorganismen an die von dem Haftvermittler bereitgestellten Haftstellen elektrostatisch oder kovalent anheften.

Eine Funktionalisierung durch eine Beschichtung bzw. Benetzung des bindungsfähigen Teilbereichs der Oberfläche erfolgt insbesondere durch das Auftragen einer ionischen Schicht. Dies ermöglicht die elektrostatische Anheftung von Mikroorganismen, wobei eine vermittelnde ionische Bindung zwischen dem bindungsfähigen Teilbereich der Oberfläche bzw. der auf den bindungsfähigen Teilbereich der Oberfläche aufgetragenen ionischen Schicht und anzuheftenden Mikroorganismen, herstellbar ist. Die ionische Schicht ist vorteilhafterweise durch einen ionischen Haftvermittler realisiert, wie beispielsweise einem Polyelektrolyt. Das Auftragen der ionischen Schicht auf den bindungsfähigen Teilbereich erfolgt durch die Benetzung mit dem ionischen Haftvermittler.

Das Auftragen einer kovalent bindenden Schicht erfolgt insbesondere durch das Auftragen eines Vernetzungsmittels als Haftvermittler, wobei das Vernetzungsmittels auf einen bindungsfähigen Teilbereich der Oberfläche aufgetragen wird. Zur Aufbringung der kovalent bindenden Schicht bzw. des Vernetzungsmittels kann beispielsweise der jeweilige bindungsfähige Teilbereich der Oberfläche zuvor derart aktiviert werden, so dass das Vernetzungsmittel eine kovalente Bindung mit diesem Teilbereich der Oberfläche des Tragelementes herstellen kann. Die Anheftung von Mikroorganismen erfolgt anschließend derart, dass diese über das Vernetzungsmittel eine kovalente Bindung mit der kovalent bindenden Schicht bzw. dem Vernetzungsmittel herstellen. Als Vernetzungsmittel eignen sich insbesondere Epoxide, wie beispielsweise Epoxysilan.

In einer weiteren Ausgestaltung der Erfindung sind die bindungsfähigen Teilbereiche der Oberfläche des Tragelementes im Wesentlichen frei von Kavitäten. In solchen Kavitäten können sich Mikroorganismen vor Sterilisationsbedingungen schützen. Eine Ursache für eine höhere Resistenz einer Populationen von Mikroorganismen auf einem Tragelement eines bekannten Bioindikators ist, dass die Oberfläche des Trägers, auf der die Mikroorganismen angeordnet sind, Unebenheiten wie insbesondere Kavitäten aufweist. Einzelne Mikroorganismen liegen geschützt in einer solchen Kavität, so dass dieser Mikroorganismus durch die äußeren Gegebenheiten der Kavität zumindest teilweise vor den während des Sterilisationsvorgangs herrschenden Sterilisationsbedingungen geschützt ist. Entsprechend ist erfindungsgemäß vorgesehen, den Teilbereich der Oberfläche des Tragelementes, auf dem Mikroorganismus bzw. eine Population von Mikroorganismen angebunden werden, derart auszugestalten, dass im Wesentlichen keine Kavitäten vorhanden sind, in denen sich Mikroorganismen vor Sterilisationsbedingungen schützen können. Ermöglicht wird dies beispielsweise durch die Verwendung eines mit dieser Eigenschaft versehenen Tragelementes, wobei eine erfindungsgemäße Ausgestaltung der Erfindung, die dies ermöglicht im Nachfolgenden angeführt ist.

Eine weitere Ausgestaltung nach einem der vorstehenden Aspekte der Erfindung sieht vor, dass die Oberfläche des Tragelementes im Wesentlichen glatt ist. Glatt im Sinne der Erfindung bedeutet, dass keinerlei Blasen oder Kuhlen als Erhebungen bzw. Vertiefungen auf der Oberfläche vorhanden sind. Beispielsweise können aus einer Schmelze gegossene Materialien, welche mit blasenfreien Verfahren gefertigt werden, verwendet werden. Zur Vermeidung beispielsweise von Blasen kann wo nötig vor dem Gießen eine Entgasung erfolgen. Ein Tragelement kann ferner mit Oberflächenverfahren geglättet werden, beispielsweise indem es hoch poliert wird.

Durch diese glatte Ausgestaltung der Oberfläche des Tragelementes ist die Oberfläche insbesondere auch frei von Kavitäten, in denen sich einzelne Mikroorganismen vor Sterilisationsbedingungen schützen können. Dies erleichtert das Ablösen von angelagerten Mikroorganismen an einem nicht-bindungsfähigen Teilbereich der Oberfläche des Tragelementes beispielsweise durch Waschverfahren. Diejenigen Mikroorganismen, die an einem funktionalisierten Teilbereich der Oberfläche des Tragelementes angeheftet sind, können durch die dauerhafte und stabile Bindung des Haftvermittlers an dem Tragelement durch das Waschverfahren nicht abgelöst werden. Dadurch kann sichergestellt werden, dass Mikroorganismen nur an dem funktionalisierten Teilbereichen durch die spezifischen Haftpositionen bzw. Haftstellen des Haftvermittlers gebunden werden.

Eine Ausgestaltung der Erfindung sieht vor, dass das Tragelement im Wesentlichen plattenförmig, kugelförmig, polyedrisch oder dergleichen ist. Dabei ist das Tragelement beispielsweise aus Glas oder aus Kunststoff oder dergleichen. Als Material für das Tragelement eignet sich beispielsweise Silikatglas oder Polycarbonat, um insbesondere das Tragelement mit den vorstehend angeführten Merkmalen der Erfindung zu realisieren.

In einer weiteren Ausgestaltung sind die bindungsfähigen Teilbereiche der Oberfläche des Tragelementes gegenüber dem nicht-bindungsfähigen Teilbereich der Oberfläche des Tragelementes erhöht. Vorzugsweise sind die bindungsfähigen Teilbereiche der Oberfläche des Tragelementes auf säulenartige Erhöhungen angeordnet. Die Erhöhung der bindungsfähigen Teilbereiche der Oberfläche des Tragelementes vermindert die Gefahr von einer Überwanderung, d.h. einer gegenseitigen Kontamination der Mikroorganismen von den jeweiligen auf der erfindungsgemäßen Vorrichtung anhaftenden Populationen von Mikroorganismen.

In einer vorteilhaften Ausgestaltung der Erfindung sind die Mikroorganismen Sporen, insbesondere Endosporen der Familie der Bacillaceae und Clostridiaceae. Vorzugsweise sind die Endosporen vom Typ Bacillus oder Clostridium. Eine Vielzahl dieser Mikroorganismen kann dabei jeweils in einer Population bzw. Kolonie bildendenden Einheit auf je einem bindungsfähigen und funktionalisierten Teilbereich der Oberfläche des Tragelementes erfindungsgemäß anheften.

Eine Ausgestaltung der erfindungsgemäßen Vorrichtung sieht eine Hülle zur Transportsicherung vor, wobei die Hülle die auf der Vorrichtung angehefteten Mikroorganismen vor einer Kontamination schützt. Die umgebende Hülle kann lösbar sein, so dass diese vor der Durchführung einer Sterilisation abgelöst werden kann oder die Hülle ist mikroporös, so dass Sterilisationsmittel die Hülle durchdringen kann, d.h. die Hülle ist für Sterilisationsmittel, wie Dampf und Wasserstoffperoxid ($H_2O_2$) durchlässig, jedoch undurchlässig für Mikroorganismen, so dass eine Kontamination der auf dem Tragelement der Vorrichtung angebundenen Mikroorganismen bzw. der Populationen von Mikroorganismen ausgeschlossen werden kann. Daher kann die umgebende Hülle auch die Funktion einer Transportsicherung bieten.

Die erfindungsgemäße Vorrichtung eignet sich dabei sowohl zur Anwendung bei einer thermischen als auch bei einer chemischen Sterilisation.

Die erfindungsgemäße Aufgabe wird ferner durch ein Verfahren zur Herstellung einer erfindungsgemäßen Vorrichtung gelöst, welches die folgenden Verfahrensschritte umfasst:

- Bereitstellen eines Tragelementes, wobei mindestens zwei Teilbereiche der Oberfläche des Tragelementes bindungsfähig sind und wobei die bindungsfähigen Teilbereiche voneinander beabstandet sind;

- Funktionalisierung der bindungsfähigen Teilbereiche der Oberfläche des Tragelementes mit einem Haftvermittler, wobei der Haftvermittler einerseits an dem bindungsfähigen Teilbereich der Oberfläche anbindet und andererseits eine durch die Größe des bindungsfähigen Teilbereichs bedingte freier Haftstellen für anzuheftende Mikroorganismen bereitstellt, so dass zwischen dem bindungsfähigen Teilbereich der Oberfläche des Tragelementes und anzuheftenden Mikroorganismen eine stabile vermittelnde Bindung herstellbar ist;

- Reinigung von Mikroorganismen, wobei an den Mikroorganismen anhaftendes überschüssiges biologisches Material entfernt wird, so dass die Mikroorganismen quasi Einzelzellcharakter aufweisen; und

- Anheftung der Mikroorganismen, wobei die gereinigten Mikroorganismen auf den mit dem Haftvermittler funktionalisierten Teilbereichen gegeben werden und die Mikroorganismen an den von dem Haftvermittler bereitgestellten Haftstellen anheften, so dass die Mikroorganismen in einer hohen Flächendichte und im Wesentlichen ohne gegenseitige Überdeckung in einer Einzelschicht angeheftet sind

Die einzelnen Verfahrensschritte des erfindungsgemäßen Verfahrens können insbesondere in der beschriebenen Art und Weise der erfindungsgemäßen Vorrichtung durchgeführt werden.

In einer Ausgestaltung der Erfindung erfolgt die Funktionalisierung wenigstens eines bindungsfähigen Teilbereichs, indem eine Bedruckung mit einem Polyelektrolyt als ionischer Haftvermittler erfolgt. Hierzu kann insbesondere eine Art Stempel, beispielsweise ein Silikonstempel für die Dauer von einigen Minuten in eine Polyelektrolyt Lösung gegeben werden und anschließend in Kontakt mit wenigstens einem aktivierten Teilbereich des Tragelementes gebracht werden. Überschüssiges Polyelektrolyt kann vorzugsweise mit einem geeigneten Mittel, wie beispielsweise Wasser, abgewaschen werden.

Die Anheftung von Mikroorganismen auf wenigstens einem funktionalisierten Teilbereich kann beispielsweise erfolgen, insbesondere durch eine elektrostatische Anbindung, beispielsweise an den zahlreichen primären und sekundären Aminogruppen der Polyelektrolyte. Beispielsweise tragen diese dissoziierbare Gruppen. Bei der Zugabe von Wasser werden diese Gruppen protoniert, wobei ein stark basisches Polykation entsteht. Das Polykation kann beispielsweise elektrostatisch an den Anionen der Oberfläche eines Mikroorganismus binden. Vorteilhafterweise ist das elektronische Potenzial an der Oberfläche der anzuheftenden Mikroorganismen hoch mit einem Zetapotenzial der Mikroorganismen von z. B. -60mV, wobei Anionen an deren Oberfläche insbesondere durch Suspendieren in deionisiertem Wasser, mit einem pH-Wert von etwa 6 erreicht wird.

Eine Ausgestaltung nach einem der vorstehenden Aspekte ist gekennzeichnet durch den folgenden Verfahrensschritt:

- Reinigung des Tragelementes, wobei sekundär angelagerte und/oder an einem nicht-bindungsfähigen Teilbereich angelagerte Mikroorganismen von dem Tragelement entfernt werden.

Hierzu eignet sich beispielsweise ein Lösungsmittel, wie 2-Propanol. Ferner kann eine Reinigung des Tragelementes alternativ oder zusätzlich in einem Ultraschallbad erfolgen.

Einer weiteren Ausgestaltung der Erfindung nach einem der vorstehenden Aspekte ist dadurch gekennzeichnet, dass die mindestens zwei bindungsfähigen Teilbereiche vor der Funktionalisierung aktiviert werden, wobei die Aktivierung vorzugsweise durch eine Plasmabehandlung erfolgt.

Vorteilhafterweise kann das Tragelement vor der Aktivierung gereinigt werden.

Durch die Plasmabehandlung wird die Oberflächenspannung an dem bindungsfähigen Teilbereich des Tragelementes erhöht. Die Plasmabehandlung kann vorzugsweise in einem Plasmaofen, wie beispielsweise einer Vakuumkammer mit Glimmentladung, erfolgen, wobei ein nicht zu aktivierender Teilbereich der Oberfläche des Tragelementes abgedeckt wird, beispielsweise durch eine Silikonschablone bzw. Lochmaske. Dies verhindert wirksam eine Aktivierung dieses abgedeckten Teilbereichs der Oberfläche des Tragelementes. Zur Aktivierung wird das Tragelement beispielsweise mit Sauerstoffplasma, vorzugsweise bei etwa 0,1 mbar für die Dauer von etwa 1 min bis etwa 10 min behandelt. Die Plasmabehandlung bewirkt, dass einige Kohlenstoffgruppen des Tragelementes oxidiert werden, wobei sich Sauerstoffatome anlagern und reaktive aktive Hydroxlgruppen entstehen.

Eine weitere Ausgestaltung der Erfindung ist dadurch gekennzeichnet, dass die Anheftung der Mikroorganismen erfolgt, indem anzuheftende Mikroorganismen im Überschuss auf den wenigstens einen funktionalisierten Teilbereich der Oberfläche des Tragelementes aufgebracht werden.

Eine weitere Ausgestaltung der Erfindung sieht vor, dass die Anheftung der Mikroorganismen erfolgt, indem anzuheftende Mikroorganismen, beispielsweise als Suspension im Überschuss auf wenigstens einen funktionalisierten Teilbereich der Oberfläche des Tragelementes aufgebracht werden. Die Bindung zwischen einem Mikroorganismus und einer von dem Haftvermittler an dem funktionalisierten Teilbereich bereitgestellten Haftstelle ist stärker, als die Bindung der Mikroorganismen untereinander. Dies erlaubt eine Anheftung der Mikroorganismen ausschließlich an den Haftstellen des Haftvermittlers an dem mit diesem funktionalisierten Teilbereich des Tragelementes. Durch die erfindungsgemäße chemische Reinigung der Mikroorganismen und dem Vorliegen von anzuheftenden Mikroorganismen mit Einzelzellcharakter kann eine hohe Flächendichte der Mikroorganismen in einer quasi Monoschicht an dem funktionalisierten Teilbereich der Oberfläche des Tragelementes erzielt werden.

Ferner wird die erfindungsgemäße Aufgabe durch die Verwendung der erfindungsgemäßen Vorrichtung zur Überprüfung einer Sterilisationswirkung und/oder als Bioindikator gelöst.

Weitere Einzelheiten, Merkmale und Vorteile der Erfindung werden nachfolgend anhand des in den Figuren dargestellten Ausführungsbeispiels näher erläutert.

**Kurze Beschreibung der Figuren**

Es zeigen

Fig. 1    eine Ausgestaltung einer erfindungsgemäßen Vorrichtung in einer perspektivischen und schematischen Darstellung;

Fig. 2    eine weitere Ausgestaltung einer erfindungsgemäßen Vorrichtung in einer vertikalen und schematischen Schnittdarstellung; und

Fig. 3    eine elektromikroskopische Aufnahme von auf einer Ausgestaltung einer erfindungsgemäßen Vorrichtung angehefteten Mikroorganismen.

**Detaillierte Beschreibung einiger beispielhafter Ausführungsformen**

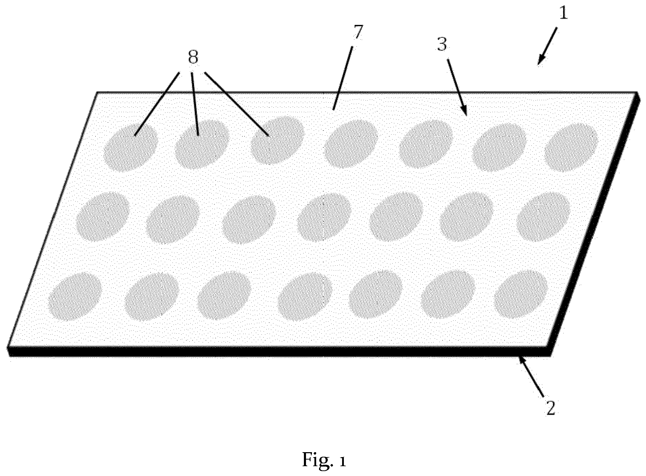

In Fig. 1 ist eine Ausgestaltung einer erfindungsgemäßen Vorrichtung 1 in einer perspektivischen und schematischen Darstellung gezeigt. An die Vorrichtung 1 können beispielsweise Mikroorganismen 9 (z.B. Sporen) zur Überprüfung einer Sterilisationswirkung angeheftet werden. Es können beispielsweise Sporen der Familie Bacillaceae oder Clostridiaceae angeheftet werden. Die Vorrichtung 1 umfasst ein Tragelement 2. Das Tragelement 2 ist beispielsweise plattenförmig ausgestaltet. Als Material für das Tragelement 2 wird beispielsweise ein Glassubstrat verwendet. Das Tragelement 2 weist 21 Mikroorganismus-Populationen 8 auf, die auf aktivierten Teilbereichen 4 auf der Oberfläche 3 des Tragelementes 2 angebunden sind. Die einzelnen bindungsfähigen Teilbereiche 4 sind jeweils voneinander in gleichmäßigen Abständen beabstandet. Die einzelnen bindungsfähigen Teilbereiche 4 weisen durch den untereinander vorliegenden physikalischen Abstand keine gegenseitigen Berührungspunkte auf. Entsprechend sind die Mikroorganismus-Populationen 8 jeweils voneinander beabstandet.

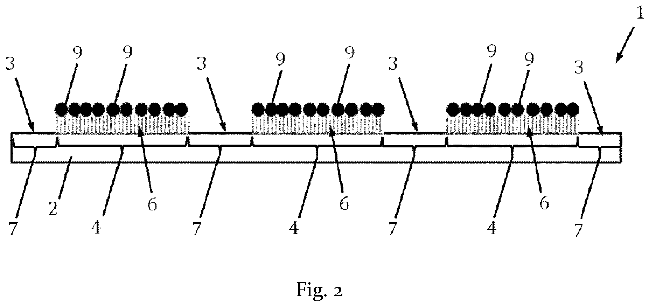

Die Vorrichtung 1 umfasst einen Haftvermittler (vgl. Bezugszeichen 6 in Fig. 2), wobei der Haftvermittler 6 jeweils auf einem bindungsfähigen Teilbereich 4 der Oberfläche 3 angeordnet ist und somit diesen bindungsfähigen Teilbereich 4 funktionalisiert. Es sind funktionalisierte Teilbereiche 5 eingerichtet und/oder ausgebildet. Der Haftvermittler 6 bedeckt insbesondere vollständig den jeweiligen bindungsfähigen Teilbereich 4, wobei der Haftvermittler 6 an dem bindungsfähigen Teilbereich 4 der Oberfläche 3 des Tragelementes 2 anbindet. Ferner stellt der Haftvermittler 6 freie Haftstellen für anzuheftende Mikroorganismen 9 (z.B. Sporen) bereit. Derart ist zwischen dem jeweiligen bindungsfähigen Teilbereich 4 der Oberfläche 3 des Tragelementes 2 und anzuheftenden Mikroorganismen 9 eine stabile vermittelnde Bindung herstellbar.

Die dargestellte Ausgestaltung einer erfindungsgemäßen Vorrichtung 1 umfasst zumindest eine Population von Mikroorganismen 8, vorliegend eine Sporenpopulation. Auf der Vorrichtung 1 ist beispielsweise jeweils eine Sporenpopulation 8 auf einem der 21 bindungsfähigen und mit dem Haftvermittler 6 funktionalisierten Teilbereiche 5 der Oberfläche 3 des Tragelementes 2 angeheftet. Die Mikroorganismen (z.B. Sporen 9) der jeweiligen Population 8 sind vorliegend chemisch gereinigt. Bei der erfindungsgemäßen chemischen Reinigung erfolgt eine Aufreinigung der Mikroorganismen (z.B. Sporen 9), bei der anhaftendes überschüssiges biologisches Material von dem Mikroorganismen (z.B. Sporen 9) entfernt wird. Derart weist ein einzelner chemisch gereinigter Mikroorganismen (z.B. Sporen 9) quasi Einzelzellcharakter auf. Die derart chemisch gereinigten Mikroorganismen (z.B. Sporen 9) werden anschließend auf einem der funktionalisierten Teilbereiche 5 angeheftet. Durch den Einzelzellcharakter der jeweiligen Mikroorganismen (z.B. Sporen 9) einer Population 8 sind die Mikroorganismen (z.B. Sporen 9) in einer hohen Flächendichte an der Vorrichtung 1 anheftbar.

Sämtliche bindungsfähigen Teilbereiche 4 des Tragelementes 2 haben im Wesentlichen die gleiche Größe und Form. Durch diese gleiche Größe und Form ist an jedem der bindungsfähigen Teilbereiche 4 im Wesentlichen die gleiche Individuenanzahl von Mikroorganismen, vorliegend Sporen 9 anheftbar. Vorliegend sind die einzelnen Mikroorganismen (z.B. Sporen 9) der jeweiligen Population 9 elektrostatisch auf erfindungsgemäße Art und Weise an dem Tragelement 2 angeheftet.

In Fig. 2 ist eine weitere Ausgestaltung einer Vorrichtung (z.B. Vorrichtung 1 nach Fig. 1) in einer vertikalen und schematischen Schnittdarstellung gezeigt.

Dargestellt sind drei in der Schnittdarstellung zu erkennende bindungsfähige (z.B. durch eine entsprechende Aktivierung des Bereichs der Oberfläche 3) Teilbereiche 4 auf der Oberfläche 3 des Tragelementes 2 der Vorrichtung 1. Die bindungsfähigen Teilbereiche 4 sind voneinander beabstandet. Zwischen den bindungsfähigen Teilbereichen 4 ist die Oberfläche 3 des Tragelementes 2 jeweils nicht aktiviert. Sie weist also zwischen den bindungsfähigen Teilbereichen 4 nicht-aktivierte Teilbereiche 7 auf, wobei dieser Bereich mit der geschweiften Klammer gekennzeichnet ist.

Auf den bindungsfähigen Teilbereichen 4 der Oberfläche 3 des Tragelementes 2 ist jeweils ein Haftvermittler 6 angeordnet bzw. angebunden, wobei die bindungsfähigen Teilbereiche 4 jeweils vollständig mit dem Haftvermittler 6 benetzt sind. Der Haftvermittler 6 bindet an den bindungsfähigen Teilbereichen 4 der Oberfläche 3 des Tragelementes 2 an. Zudem stellt der Haftvermittler 6 freie Haftstellen bereit, an welchen Mikroorganismen (z.B. Sporen 9) anheften können. Schematisch ist der Haftvermittler durch senkrecht verlaufende Linien dargestellt, die verdeutlichen sollen, dass der Haftvermittler 6 eine stabile vermittelnde Bindung zwischen Mikroorganismen (z.B. Sporen 9) und einem bindungsfähigen Teilbereich 4 der Oberfläche des Tragelementes herstellen kann. Der Haftvermittler 6 bindet also einerseits an dem bindungsfähigen Teilbereich 4 der Oberfläche 3 des Tragelementes 2 an, und stellt andererseits freie Haftstellen für Mikroorganismen (z.B. Sporen 9) bereit.

Schematisch sind lediglich einige an dem Haftvermittler 6 angeheftete Mikroorganismen (z.B. Sporen 9) dargestellt. Real sind etwa $10^6$ Mikroorganismen (z.B. Sporen 9) als eine Population 8 pro bindungsfähigen und mit dem Haftvermittler 6 funktionalisierten Teilbereich 5 an der Oberfläche 3 des Tragelementes 2 angeheftet. Die Anheftung der Mikroorganismen (z.B. Sporen 9) einer Ppopulation 8 erfolgt im Wesentlichen ohne gegenseitige Überdeckung der Sporen, wie in Fig. 2 ebenfalls schematisch dargestellt ist. Die Mikroorganismen (z.B. Sporen 9) sind also in einer Einzellage bzw. quasi Monoschicht an der Vorrichtung 1 angeheftet.

Erfindungsgemäß wird dies zum einen durch die chemische Reinigung der Mikroorganismen (z.B. Sporen 9) ermöglicht, wobei die Mikroorganismen (z.B. Sporen 9) vor der Anheftung entsprechend vorbereitet werden. Bei der chemischen Reinigung der Mikroorganismen (z.B. Sporen 9) wird an diesen anhaftendes biologisches Material, insbesondere Zelltrümmer entfernt. Dieses ist überschüssig und wird zur Überprüfung einer Sterilisationswirkung nicht benötigt. Die intensive chemische Reinigung bereitet anzuheftende Mikroorganismen (z.B. Sporen 9) derart vor, dass diese einen Einzelzellcharakter aufweisen. Der Vorteil hiervon ist, dass die Bindung der Mikroorganismen (z.B. Sporen 9) zu dem funktionalisierten Teilbereich 5 des Tragelement 2 höher ist, als eine mögliche Bindung der Mikroorganismen (z. B. Sporen 9) zu einem nicht-bindungsfähigen Teilbereich 7 des Tragelementes 2 und/oder zu weiteren Mikroorganismen (z.B. Sporen 9). Insbesondere eine gegenseitige Bindung von Mikroorganismen (z.B. Sporen 9) untereinander kann zu einem Verklumpen der Mikroorganismen (z.B. Sporen 9) führen, wobei sich die Mikroorganismen bei einer Exposition gegenüber Sterilisationsbedingungen gegenseitig schützen können.

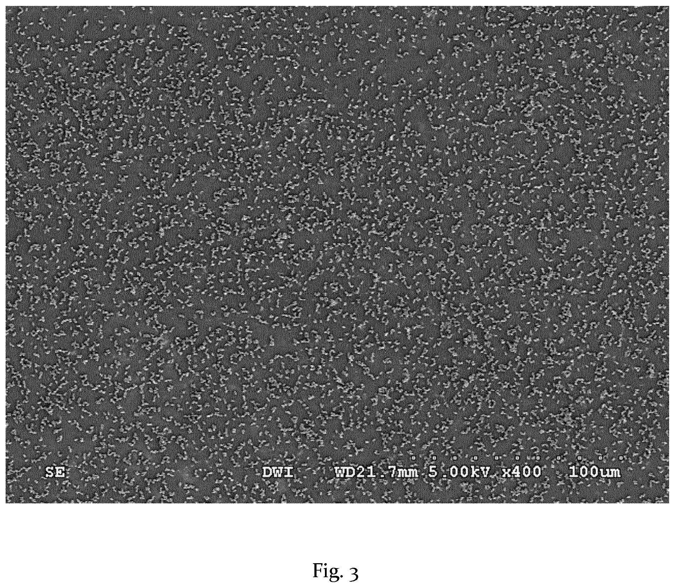

In Fig. 3 ist eine elektromikroskopische Aufnahme von auf einer Ausgestaltung einer erfindungsgemäßen Vorrichtung angehefteten Mikroorganismen dargestellt. Die dargestellten Mikroorganismen sind vom Stamm Geobacillus stearothermophilus ATCC 7953, und wurden auf einer erfindungsgemäßen Vorrichtung mittels eines ionischen Haftvermittlers angeheftet nach wenigstens einem der vorstehenden Aspekte auf einem Tragelement angeheftet. Eine exemplarische Auszählung und Hochrechnung der angehefteten Mikroorganismen ergab 1,9 x $10^5$ kbE/mm$^2$ (kbE: kolonie bildende Einheit, entspricht der Anzahl von Mikroorganismen auf einer Fläche von 1 mm$^2$). Ferner ist zu erkennen, dass die angehefteten Mikroorganismen in einer hohen Flächendichte und im Wesentlichen ohne gegenseitige Überdeckung in einer Einzelschicht angeheftet sind.

Die in Fig. 3 dargestellten Mikroorganismen wurden vor deren Anheftung auf einer Vorrichtung hoch gereinigt, wobei die folgenden Reinigungsschritte zur Erzielung der hohen Aufreinigung in der nachstehend ausgeführten Art und Weise erzielt wurde:

- Herstellung Kaliumphosphatpuffer (3 M):

$$16,89 \text{ g } KH_2PO_4 + 30,66 \text{ g } K_2HPO_4 \text{ auf } 100 \text{ mL WFI, pH} = 7,1.$$

- Herstellung System Y:

$$11,18 \text{ g PEG } 4000 + 34,1 \text{ mL Phosphatpuffer}$$

- Von der Sporensuspension etwa 10 ml in System Y geben.

- In Falcon Tubes 15 min Schütteln, anschließend 2 min bei 1500 rcf zentrifugieren

- Oberste 2/3 der oberen Phase entnehmen

- PEG in dieser Phase durch Zentrifugation durch WFI ersetzen (3 x waschen; 6000 rcf, 30 min, 10°C).

Der Vorteil der erfindungsgemäßen Lösung gegenüber den bekannten Vorrichtungen ist, dass durch die Anheftung von mindestens zwei Populationen von Mikroorganismen, also multiplen Populationen auf nur einem Trageelement eine gemeinsame Exposition dieser multiplen Populationen gegenüber Sterilisationsbedingungen möglich ist. Dabei sind die herrschenden Sterilisationsbedingungen nicht nur für die einzelnen Mikroorganismen einer Population nahezu identisch, sondern auch für jeweilige Populationen von Mikroorganismen durch die sehr nahe räumliche Anordnung dieser zueinander. Die erfindungsgemäße Anbindung von Mikroorganismen auf dem Trageelement erlaubt, die Belastung durch Mikroorganismen, die im Prozess anforderungsgemäß inaktiviert werden sollen, prozessentsprechend zu variieren. Höhere Resistenz oder eine größere Zahl der eingesetzten Mikroorganismen erlaubt die aussagekräftige Prüfung höher wirksamer Prozesse, eine höhere Anzahl an Populationen je Träger ermöglicht eine genauere statistische Auswertung.

Die in dieser Spezifikation beschriebenen beispielhaften Ausführungsformen / Ausführungsbeispiele sollen sowohl einzeln als auch in allen Kombinationen miteinander offenbart verstanden werden. Insbesondere soll auch die Beschreibung eines von einer Ausführungsform umfassten Merkmals - sofern nicht explizit gegenteilig erklärt - vorliegend nicht so verstanden werden, dass das Merkmal für die Funktion des Ausführungsbeispiels unerlässlich oder wesentlich ist. Die Abfolge der in dieser Spezifikation geschilderten Verfahrensschritte in den einzelnen Ablaufdiagrammen ist nicht zwingend, alternative Abfolgen der Verfahrensschritte sind denkbar. Die Verfahrensschritte können auf verschiedene Art und Weise implementiert werden, so ist eine Implementierung in Software (durch Programmanweisungen), Hardware oder eine Kombination von beidem zur Implementierung der Verfahrensschritte denkbar. In den Patentansprüchen verwendete Begriffe wie "umfassen", "aufweisen", "beinhalten", "enthalten" und dergleichen schließen weitere Elemente oder Schritte nicht aus. Unter die Formulierung "zumindest teilweise" fallen sowohl der Fall "teilweise" als auch der Fall "vollständig". Die Formulierung "und/oder" soll dahingehend verstanden werden, dass sowohl die Alternative als auch die Kombination offenbart sein soll, also "A und/oder B" bedeutet "(A) oder (B) oder (A und B)". Eine Mehrzahl von Einheiten, Personen oder dergleichen bedeutet im Zusammenhang dieser Spezifikation mehrere Einheiten, Personen oder dergleichen. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

Die in den Figuren dargestellten und beschriebenen Ausführungsbeispiele dienen lediglich der Erläuterung der Erfindung und sind für diese nicht beschränkend.

BEZUGSZEICHENLISTE

1 Vorrichtung
2 Tragelement
3 Oberfläche des Tragelementes
4 bindungsfähiger Teilbereich
5 funktionalisierter Teilbereich
6 Haftvermittler
7 nicht-bindungsfähiger Teilbereich
8 Mikroorganismus-Population
9 Mikroorganismus

**Patentansprüche**

1. Vorrichtung (1) zur Überprüfung einer Sterilisationswirkung, umfassend:

ein Tragelement (2), wobei mindestens zwei Teilbereiche (4) der Oberfläche (3) des Tragelementes (2) bindungsfähig sind und wobei die bindungsfähigen Teilbereiche (4) voneinander beabstandet sind,
einen Haftvermittler (6), wobei die bindungsfähigen Teilbereiche (4) der Oberfläche (3) durch die Anordnung des Haftvermittlers (6) an diesen funktionalisierbar sind, wobei der Haftvermittler (6) einerseits an dem bindungsfähigen Teilbereich (4) der Oberfläche (3) anbindet und andererseits durch die jeweilige Größe des bindungsfähigen Teilbereichs (4) bedingt freie Haftstellen für anzuheftende Mikroorganismen (9) bereitstellt, so dass zwischen dem bindungsfähigen Teilbereich (4) der Oberfläche (3) des Tragelementes (2) und anzuheftenden Mikroorganismen (9) eine stabile vermittelnde Bindung herstellbar ist, und
eine Population (8) von Mikroorganismen, wobei die Mikroorganismen (9) der Population (8) gereinigt sind, wobei an den Mikroorganismen (9) anhaftendes überschüssiges biologisches Material entfernt ist und die jeweiligen Mikroorganismen (9) quasi Einzelzellcharakter aufweisen,

wobei jeweils eine gereinigte Population von Mikroorganismen (8) an einem der funktionalisierten Teilbereiche (5) der Oberfläche (3) derart anheftbar ist, dass die Mikroorganismen (9) in einer hohen Flächendichte und im Wesentlichen ohne gegenseitige Überdeckung in einer Einzelschicht angeheftet sind.

2. Vorrichtung (1) nach Anspruch 1, **dadurch gekennzeichnet, dass** die hohe Flächendichte der auf den funktionalisierten Teilbereichen (5) der Oberfläche (3) des Tragelementes (2) anheftbaren Mikroorganismen (9) einem Wert entspricht, dessen Standardabweichung etwa 10% oder weniger bezogen auf eine Flächendichte von etwa $10^6$ Mikroorganismen pro 5 mm$^2$ beträgt.

3. Vorrichtung (1) nach Anspruch 1 oder Anspruch 2, **dadurch gekennzeichnet, dass** die Anheftung von Mikroorganismen (9) in der Einzelschicht derart erfolgt, wobei etwa 10% oder weniger der Mikroorganismen sich gegenseitig überdecken.

4. Vorrichtung (1) nach einem der Ansprüche 1 bis 3, **dadurch gekennzeichnet, dass** die mindestens zwei bindungsfähigen Teilbereiche (4) der Oberfläche (3) des Tragelementes (2) aktiviert sind.

5. Vorrichtung (1) nach einem der Ansprüche 1 bis 4, **dadurch gekennzeichnet, dass** die jeweilige Fläche der bindungsfähigen Teilbereiche (4) auf der Oberfläche (3) des Tragelementes (2) im Wesentlichen gleich groß ist.

6. Vorrichtung (1) nach einem der Ansprüche 1 bis 5, **dadurch gekennzeichnet, dass** der Haftvermittler (6) elektrostatisch oder kovalent an den bindungsfähigen Teilbereichen (4) der Oberfläche (3) des Tragelementes (2) anbindet und anzuheftende Mikroorganismen (9) an die von dem Haftvermittler (6) bereitgestellten Haftstellen elektrostatisch oder kovalent anheften.

7. Vorrichtung (1) nach einem der Ansprüche 1 bis 6, **dadurch gekennzeichnet, dass** die bindungsfähigen Teilbereiche (4) der Oberfläche (3) des Tragelementes (2) im Wesentlichen frei von Kavitäten sind.

8. Vorrichtung (1) nach einem der Ansprüche 1 bis 7, **dadurch gekennzeichnet, dass** das Tragelement (2) im Wesentlichen plattenförmig, kugelförmig, polyedrisch oder dergleichen ist.

9. Vorrichtung (1) nach einem der Ansprüche 1 bis 8, **dadurch gekennzeichnet, dass** die bindungsfähigen Teilbereiche (4) der Oberfläche (3) des Tragelementes (2) gegenüber dem nicht-bindungsfähigen Teilbereich (7) der Oberfläche (3) des Tragelementes (2) erhöht sind.

10. Vorrichtung (1) nach einem der Ansprüche 1 bis 9, **dadurch gekennzeichnet, dass** die Mikroorganismen (9) Sporen, insbesondere Endosporen der Familie Bacillaceae oder Clostridiaceae sind.

11. Verfahren zur Herstellung einer Vorrichtung (1) nach einem der Ansprüche 1 bis 10, umfassend die folgenden Verfahrensschritte:

   - Bereitstellen eines Tragelementes (2), wobei mindestens zwei Teilbereiche (4) der Oberfläche (3) des Tragelementes (2) bindungsfähig sind und wobei die bindungsfähigen Teilbereiche (4) voneinander beabstandet sind;
   - Funktionalisierung der bindungsfähigen Teilbereiche (4) der Oberfläche (3) des Tragelementes (2) mit einem Haftvermittler (6), wobei der Haftvermittler (6) einerseits an dem bindungsfähigen Teilbereich (4) der Oberfläche (3) anbindet und andererseits eine durch die Größe des bindungsfähigen Teilbereichs (4) bedingte freier Haftstellen für anzuheftende Mikroorganismen (9) bereitstellt, so dass zwischen dem bindungsfähigen Teilbereich (4) der Oberfläche (3) des Tragelementes (2) und anzuheftenden Mikroorganismen (9) eine stabile vermittelnde Bindung herstellbar ist;
   - Reinigung von Mikroorganismen (9), wobei an den Mikroorganismen (9) anhaftendes überschüssiges biologisches Material entfernt wird, so dass die Mikroorganismen (9) quasi Einzelzellcharakter aufweisen; und
   - Anheftung der Mikroorganismen (9), wobei die gereinigten Mikroorganismen (9) auf den mit dem Haftvermittler (6) funktionalisierten Teilbereichen (5) gegeben werden und die Mikroorganismen (9) an den von dem Haftvermittler (6) bereitgestellten Haftstellen anheften, so dass die Mikroorganismen (9) in einer hohen Flächendichte und im Wesentlichen ohne gegenseitige Überdeckung in einer Einzelschicht angeheftet sind

12. Verfahren nach Anspruch 11, **gekennzeichnet durch** den folgenden Verfahrensschritt:

- Reinigung des Tragelementes (2), wobei sekundär angelagerte und/oder an einem nicht-bindungsfähigen Teilbereich (7) angelagerte Mikroorganismen (9) von dem Tragelement (2) entfernt werden.

13. Verfahren nach Anspruch 11 oder Anspruch 12, **dadurch gekennzeichnet, dass** die mindestens zwei bindungsfähigen Teilbereiche vor der Funktionalisierung aktiviert werden, wobei die Aktivierung vorzugsweise durch eine Plasmabehandlung erfolgt.

14. Verfahren nach einem der Ansprüche 11 bis 13, **dadurch gekennzeichnet, dass** die Anheftung der Mikroorganismen erfolgt, indem anzuheftende Mikroorganismen (9) im Überschuss auf den wenigstens einen funktionalisierten Teilbereich (5) der Oberfläche (3) des Tragelementes (2) aufgebracht werden.

15. Verwendung der Vorrichtung (1) nach einem der Ansprüche 1 bis 10 zur Überprüfung einer Sterilisationswirkung und/oder als Bioindikator.

Fig. 1

Fig. 2

SE              DWI     WD21.7mm 5.00kV x400   100um

Fig. 3

**Europäisches Patentamt**
**European Patent Office**
**Office européen des brevets**

# EUROPÄISCHER RECHERCHENBERICHT

**Nummer der Anmeldung**

EP 17 20 7058

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 014 762 A1 (NGK INSULATORS LTD [JP]) 14. Januar 2009 (2009-01-14) * Absätze [0010], [0012], [0014], [0041]; Abbildung 1 * ----- | 1-15 | INV. A61L2/28 C12M1/34 C12Q1/22 |
| X | WO 97/05274 A1 (MINNESOTA MINING & MFG [US]) 13. Februar 1997 (1997-02-13) * Zusammenfassung; Abbildung 1 * * Seite 5, Absatz 2 * * Seite 8, Zeile 15 - Seite 9, Zeile 5; Abbildung 3 * ----- | 1-15 | |
| X | WO 2015/200063 A1 (3M INNOVATIVE PROPERTIES CO [US]) 30. Dezember 2015 (2015-12-30) * Zusammenfassung; Abbildung 1 * ----- | 1,11 | |
| A | WO 2012/012055 A2 (AMERICAN STERILIZER CO [US]; FRANCISKOVICH PHILLIP P [US]; CREGGER TRI) 26. Januar 2012 (2012-01-26) * Seite 18, Zeile 16 - Seite 19, Zeile 3; Abbildung 9 * ----- | 1-15 | |

**RECHERCHIERTE SACHGEBIETE (IPC)**

A61L
C12M
C12Q

Der vorliegende Recherchenbericht wurde für alle Patentansprüche erstellt

| Recherchenort | Abschlußdatum der Recherche | Prüfer |
|---|---|---|
| München | 11. Mai 2018 | Fischer, Michael |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

.............................................................................................

& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 20 7058

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-05-2018

| Im Recherchenbericht angeführtes Patentdokument | Datum der Veröffentlichung | Mitglied(er) der Patentfamilie | Datum der Veröffentlichung |
|---|---|---|---|
| EP 2014762 A1 | 14-01-2009 | EP 2014762 A1 | 14-01-2009 |
| | | JP WO2007119857 A1 | 27-08-2009 |
| | | US 2009068716 A1 | 12-03-2009 |
| | | WO 2007119857 A1 | 25-10-2007 |
| WO 9705274 A1 | 13-02-1997 | AU 6340396 A | 26-02-1997 |
| | | DE 69619395 D1 | 28-03-2002 |
| | | DE 69619395 T2 | 17-10-2002 |
| | | EP 0863994 A1 | 16-09-1998 |
| | | JP H11510051 A | 07-09-1999 |
| | | US 5922592 A | 13-07-1999 |
| | | US 6096533 A | 01-08-2000 |
| | | WO 9705274 A1 | 13-02-1997 |
| WO 2015200063 A1 | 30-12-2015 | CN 106461562 A | 22-02-2017 |
| | | JP 2017534691 A | 24-11-2017 |
| | | US 2017131442 A1 | 11-05-2017 |
| | | WO 2015200063 A1 | 30-12-2015 |
| WO 2012012055 A2 | 26-01-2012 | AU 2011280113 A1 | 21-02-2013 |
| | | AU 2014224078 A1 | 02-10-2014 |
| | | CA 2805166 A1 | 26-01-2012 |
| | | CA 2962159 A1 | 26-01-2012 |
| | | CN 103119173 A | 22-05-2013 |
| | | CN 104263805 A | 07-01-2015 |
| | | EP 2596120 A2 | 29-05-2013 |
| | | EP 2746401 A2 | 25-06-2014 |
| | | ES 2524890 T3 | 15-12-2014 |
| | | ES 2606309 T3 | 23-03-2017 |
| | | JP 5789299 B2 | 07-10-2015 |
| | | JP 6257499 B2 | 10-01-2018 |
| | | JP 2013530723 A | 01-08-2013 |
| | | JP 2015037432 A | 26-02-2015 |
| | | MX 336368 B | 18-01-2016 |
| | | MX 342038 B | 12-09-2016 |
| | | US 2012021406 A1 | 26-01-2012 |
| | | US 2013217001 A1 | 22-08-2013 |
| | | US 2014162307 A1 | 12-06-2014 |
| | | WO 2012012055 A2 | 26-01-2012 |

EPO FORM P0461

Für nähere Einzelheiten zu diesem Anhang : siehe Amtsblatt des Europäischen Patentamts, Nr.12/82